Europäisches Patentamt

European Patent Office

Office européen des brevets

(19)

(11) Veröffentlichungsnummer: **0 086 996**
**B1**

(12) **EUROPÄISCHE PATENTSCHRIFT**

(45) Veröffentlichungstag der Patentschrift:
**11.09.85**

(51) Int. Cl.⁴: **D 04 H  1/46,** D 04 H  13/00,
A 61 H  33/04

(21) Anmeldenummer: **83100848.7**

(22) Anmeldetag: **29.01.83**

(54) Verfahren zur Herstellung von faserverstärkten, flächigen Körpern.

(30) Priorität: **29.01.82  CH 549/82**

(43) Veröffentlichungstag der Anmeldung:
**31.08.83 Patentblatt 83/35**

(45) Bekanntmachung des Hinweises auf die Patenterteilung:
**11.09.85 Patentblatt 85/37**

(84) Benannte Vertragsstaaten:
**AT BE CH DE FR GB IT LI LU NL SE**

(56) Entgegenhaltungen:
**EP - A - 0 071 209**
**CH - A - 629 137**
**DE - A - 1 435 762**
**DE - A - 1 560 651**
**DE - A - 2 742 030**
**DE - B - 2 701 469**
**FR - A - 1 394 567**
**FR - A - 1 530 425**
**FR - A - 2 198 836**
**FR - A - 2 248 937**
**FR - A - 2 412 405**
**GB - A - 440 064**
**US - A - 2 094 148**
**US - A - 2 314 162**
**US - A - 2 920 373**
**US - A - 3 040 740**

(73) Patentinhaber: **Tesch, Günter Horst, Avenue
Jean-Marie-Musy 15, CH-1700 Fribourg (CH)**

(72) Erfinder: **Tesch, Günter Horst, Avenue
Jean-Marie-Musy 15, CH-1700 Fribourg (CH)**

(74) Vertreter: **Lesser, Karl-Bolko, Dipl.-Ing., European
Patent Attorney Johanneskirchnerstrasse 149a,
D-8000 München 81 (DE)**

(56) Entgegenhaltungen: (Fortsetzung)
**US - A - 3 415 713**
**US - A - 3 448 502**
**US - A - 4 199 635**

**Beschreibung**

Die Erfindung betrifft ein Verfahren zur Herstellung von faserverstärkten, flächigen Körpern gemäß Oberbegriff des Anspruches 1.

In der DE-A-3 019 917 wird vorgeschlagen, Wasser enthaltende, fließfähige Schichten, wie zementschlammartige, insbesondere Gips enthaltende Massen dadurch mit Fasern zu verstärken, daß man diese Wasser enthaltende Schicht zwischen zwei durchlässige Bahnen bringt und diesen Schichtkörper so zwischen zwei Auflageflächen in Vibration versetzt, daß die schlammartige Masse die beiden außenliegenden Bahnen durchdringt. Der so gebildete Schichtkörper, der dort insbesondere einen Gipsschlamm enthält, härtet danach zu einer faserverstärkten Gipsplatte aus. Die durchlässige Bahn besteht dort bevorzugt aus Glasfasern, die gewebt oder gewirkt vorliegen. Die Glasfasern können aber auch durch ein geeignetes Kunstharz miteinander verbunden sein.

Weiterhin wird in der DE-A-3 019 917 unter Verweis auf die GB-A-772 581 beschrieben, ein Glasfasergewebe durch einen Gipsschlamm hindurchzuführen, auf dieses getränkte Glasfasergewebe eine Schicht aus Gipsschlamm aufzubringen, auf die ein zweites getränktes Glasfasergewebe aufgelegt wird, worauf der Schichtkörper aushärtet.

Gemäß einem weiteren der DE-A-3 019 917 entnehmbaren Verfahren wird ein Kern aus Gipsschlamm auf einer Seite einem Glasfaservlies oder Carton und auf der anderen Seite mit einem Glasfasergewebe oder einem Streifen aus Glasfaservlies, Carton, Folie oder Papier bekleidet.

Allen der DE-A-3 019 917 entnehmbaren Verfahren ist gemeinsam, daß der Schichtkörper einen inneren Zusammenhalt erst durch das Aushärten des Gipses, der ein Bindemittel darstellt, erhält, weshalb der Schichtkörper bis zum endgültigen Aushärten der schlammigen Masse durch Auflagebänder oder dergleichen gestützt werden muß. Ein dreidimensionales Verformen im noch feuchten Zustand der Kernschicht ist nicht möglich, da sich die Schichten untereinander verschieben, bzw. das Material der Kernschicht in dieser verungleichmäßigt wird.

Die Handhabung eines solchen Körpers wird dadurch erschwert, weshalb solche gemäß dem bekannten Verfahren hergestellte, eine Wasser enthaltende, fließfähige Kernschicht aufweisende Körper bis zum Aushärten derselben auf einer ebenen Trag- und Stützfläche aufliegen müssen.

Die vorgenannten aus der DE-A-3 019 917 bekannten Körper härten nun aufgrund chemischer Vorgänge innerhalb einer bestimmten Zeit aus, und bilden dann ein steifes Produkt, welches in der ausgehärteten Form gehandhabt werden kann.

Es gibt nun Wasser enthaltende Gegenstände, die durch Fasern verstärkt werden sollen, bei denen jedoch keine durch die Anwesenheit von Wasser ablaufende chemische Reaktion stattfindet.

Der Erfindung liegt die Aufgabe zugrunde, ein gattungsgemäßes Verfahren zu schaffen, mit dem auch einen eigenen inneren Zusammenhalt aufweisende Körper hergestellt werden können, deren Kernschicht nicht aushärtet.

Diese Aufgabe wird durch den Gegenstand des Anspruches 1 gelöst. Zur Lösung dieser Aufgabe wird das aus der Textiltechnik bekannte Verfahren des Vernadelns herangezogen.

Beim sogenannten Vernadeln werden aus einer auf eine andere Schicht aufgelegten, faserhaltigen Schicht mittels mit Widerhaken versehener Nadeln Einzelfasern oder Faserbüschel in diese andere Schicht hineingestochen, in der sie beim Zurückziehen der Nadeln stecken bleiben und dadurch die Verbindung der faserhaltigen Schicht mit der anderen Schicht herbeiführen. Voraussetzung zur Durchführung der Technik des Vernadelns ist somit das Vorhandensein einer Schicht aus »aktiv nadelfähigen Stoffen«, d. h., einer Schicht, welche aus zur Durchführung des Nadelprozesses heranziehbaren, faserförmigen Gebilden besteht oder derartige Gebilde enthält. Die andere Schicht, in die die aktiv nadelbaren Fasern eingebracht werden, muß mindestens passiv nadelbar sein, d. h., sie muß die in sie eingestochenen Fasern halten können.

Eine solche passiv nadelbare Schicht kann selbst aktiv nadelfähig sein, passiv nadelbare Schichten können aber auch bekannterweise durch Kunststoffolien, Papier oder dergleichen gebildet sein. Es hat sich nun überraschenderweise herausgestellt, daß auch Wasser enthaltende, fließfähige Massen, wie schlammiger Moorbrei, zerkleinertes, mit Wasser getränktes Papier, oder Rohkeramikbrei oder dergleichen passiv nadelfähig sind. Durch das Vernadeln des aus den einzelnen Schichten bestehenden, Wasser enthaltenden Schichtkörpers können sehr schnell eine Vielzahl von Haltefasern in relativ großer Dichte in den Schichtkörper eingebracht werden. Die drei Schichten werden untereinander gehalten, insbesondere wird die Wasser enthaltende Kernschicht am Austreten aus dem Schichtkörper und am Verschieben innerhalb desselben gehindert.

Der so gebildete, mattenförmige Schichtkörper hat einen eigenen inneren Zusammenhalt und kann nun auch ohne Trag- und/oder Stützfläche — freischwebend — gehandhabt werden.

Das in der Kernschicht vorhandene Wasser dient als Quellmittel für die Kernschichtmasse, wie Papierstreifen oder -schnitzel, Moorpartikel oder dergleichen, wodurch diese aufgequollen vorliegen und sehr geschmeidig sind. Darüber hinaus dient das Wasser auch als Gleitmittel für die Vernadelungsnadeln und die hindurchzustechenden Haltefasern. Durchgeführte Versuche haben gezeigt, daß beim Vernadeln von aufeinandergelegten trockenen Papierstreifen bzw. -schnitzeln, aber auch von trockenen Torfpartikeln die Kernschicht äußerst dünn gewählt werden muß, da bei größerer Dicke derselben die Vernadelungsnadeln Schwierigkeiten haben, durch diese Kernschicht hindurchzudringen und sehr schnell brechen. Bei Wasser enthaltenden Massen trat ein Nadelbruch, der im übrigen auch in der Textilindustrie gelegentlich vorkommt, fast nie auf.

Als aktiv nadelfähige Fasern können herkömmliche Synthesefasern aus Polyester, Polyamid, Polypropylen oder dergleichen oder natürliche Fasern, wie Sisal, Leinen, Baumwolle oder dergleichen verwendet werden, die Auswahl ist hier auch in bezug auf die zu vernadelnde Kernschicht zu wählen. Die zweite Außenschicht, die mindestens passiv vernadelbar sein muß, kann aus denselben Fasern bestehen, es kann dafür aber auch ein Gewebe, ein Spunbond, Kunststoffolien oder dergleichen verwendet werden.

Beim Vernadeln können nun gemäß einer Ausführungsform die Haltefasern unter einem Winkel von kleiner als 90° zur Erstreckungsebene des Körpers eingestoßen werden, wobei bevorzugt die Haltefasern sowohl von der Deckschicht, als auch von der Unterlagsschicht her eingestoßen werden und dann zueinander windschief ausgerichtet sind. Dadurch läßt sich ein solcher Körper zusätzlich verstärken.

Die Vernadelung der drei Schichten bringt es mit sich, daß die Wasser enthaltende Masse der Kernschicht nicht nur zwischen den beiden Außenschichten gehalten wird, sondern auch an einem wesentlichen Verschieben in der Erstreckungsebene des Körpers gehindert wird. Dadurch ist es möglich, in den vernadelten und dadurch verfestigten Körper quer zu seiner Erstreckungsebene Öffnungen, wie Ausstanzungen, Schlitze oder dergleichen anzubringen, ohne daß wesentliche Mengen der Kernschicht aus dem Körper austreten können, da diese Masse von den Haltefasern zurückgehalten wird.

Ist der Körper mit einer Vielzahl von in zueinander parallelen Reihen angeordneten Schlitzen versehen, wobei die Schlitze benachbarter Reihen gegeneinander versetzt angeordnet sind, so kann der vernadelte Körper quer zur Erstreckungsrichtung gedehnt werden. Ein solcher Körper ist sehr flexibel, wodurch er sich besonders leicht auch größeren Unebenheiten eines anderen Gegenstandes, auf dem er angebracht wird oder auf den er aufgelegt wird, anpaßt.

Dieses »Dehnungsvermögen« ist z. B. dann von Vorteil, wenn der Körper einen Moorbrei enthält und als Moorpackung auf verschiedene Teile des menschlichen Körpers aufgelegt werden soll. Ein und dieselbe Moorpackungs-Ausbildung kann dann als Gesichtsmaske oder auch als Auflage auf den Rücken verwendet werden. Während ein solcher Schichtkörper auf dem Rücken relativ eben zu liegen kommt, ist es bei der Verwendung als Gesichtsmaske notwendig, daß der Körper sehr flexibel ist und sich der Form des Gesichtes anpaßt.

Durch dieses Versehen des Schichtkörpers mit Öffnungen, wie Schlitzen, wird somit die Formbarkeit des Körpers sehr erleichtert.

Es ist auch möglich, aus dem vernadelten Schichtkörper einzelne Öffnungen herauszustanzen, um so z. B. bei einer Gesichtsmaske Ausschnitte für die Augen, den Mund und die Nasenlöcher zu erhalten.

Weist der genadelte Schichtkörper mehrere, miteinander verbundene und zwischen sich einen Winkel einschließende Öffnungen oder Schlitze auf, so lassen sich die zwischen diesen bzw. Abschnitten derselben befindlichen laschenförmigen Teile des Schichtkörpers aus der Ebene des Körpers herausbiegen. Solche herausgebogenen Laschen können als Haltelaschen dienen, wobei z. B. in ihnen angebrachte Öffnungen als Halteösen dienen.

Dieses Herausbiegen von Laschen kann auch durch Herausstanzen derselben vorgenommen werden, d. h., das Einbringen von Öffnungen und das Herausbiegen der Laschen wird in einem Arbeitsgang durchgeführt.

Aufgrund des inneren Zusammenhaltes des genadelten, Wasser enthaltenden Schichtkörpers ist es insbesondere dann, wenn dieser mit Öffnungen oder Schlitzen versehen ist, möglich, einen solchen Schichtkörper tief zu ziehen.

Zwei oder mehrere vollflächige Schichtkörper können auch aufeinanderverlegt miteinander vernadelt werden, wodurch ein Schichtkörper beliebiger Dicke erhalten werden kann, der insgesamt einen eigenen inneren Zusammenhalt aufweist.

Ähnlich können auch sandwichartig solche, Wasser enthaltende Schichtkörper auf andere Gegenstände, wie Schaumstoffplatten oder dergleichen aufgenadelt werden. Solche Schaumstoffplatten können aber auch von vornherein als passiv nadelbare Unterlagsschicht zur Anwendung gelangen.

Gemäß einer besonderen Ausführungsform der Erfindung wird der noch unvernadelte, Wasser enthaltende Schichtkörper geformt, z. B. als Gesichtsmaske der Gesichtsform angepaßt, und erst in dieser Form vernadelt.

Es wurde oben schon angesprochen, daß schlammiger Moorbrei als Kernschicht aufgelegt wird, wobei, wenn dieser Schichtkörper als auf den menschlichen Körper aufzulegende Moorpackung verwendet werden soll, es sich empfiehlt, die passiv nadelbare Unterlagsschicht durch eine Kunststoffolie zu bilden. Gibt man in diesen Moorbrei vor dem Vernadeln noch inerte Partikel, wie z. B. Sandkörner, die gegenüber dem Moorbrei ein höheres spezifisches Gewicht aufweisen, so kann eine solche Packung auch für Moorbäder benutzt werden, indem diese Packung am Boden einer mit Wasser gefüllten Wanne zu liegen kommt. Während nun die im Moor enthaltenen Wirkstoffe von dem die Packung durchdringenden Wasser aus der Packung mitgenommen werden, bleiben die groben Bestandteile des Moores innerhalb des Schichtkörpers gebunden. Nach Gebrauch kann die Moormasse mit den groben Partikeln der Wanne entnommen werden, und nur die feinen Partikel der Wanne entnommen werden, und nur die feinen Partikel gelangen in das Abwasser. Die Abwasserleitungen brauchen deshalb, um Verstopfungen durch das Moor zu verhindern, nicht besonders ausgebildet werden, weshalb sich diese Moorpackungen insbesondere für den privaten Anwendungsbereich eignen.

Der nach dem Vernadeln in Form einer Bahn vorliegende, Moorbrei enthaltende Schichtkörper wird in einzelne Flächengebilde aufgeteilt und diese dann eine Moorpackung bildende Flächengebilde luft- und wasserdicht verpackt.

Gemäß einer anderen Ausführungsform der Erfindung wird als Kernschicht zerkleinertes, mit Wasser getränktes Papier, insbesondere Altpapier verwendet. Während bisher für die Wiederverwendung des Altpapieres dieses in der Regel soweit aufgeschlossen werden muß, daß wieder einzelne Zellulosefasern vorliegen, ist es mit dem erfindungsgemäßen Verfahren möglich, auch Papierschnitzel oder -streifen einer neuen Verwendung zuzuführen. Beim Vernadeln von wassergetränktem Papier dient das Wasser im wesentlichen nur dazu, das Vernadeln auch dikker Schichten zu ermöglichen. Dem Wasser kann aber vor dem Tränken des Papiers auch noch Leim zugegeben werden. In diesem Falle kommt dem Wasser auch die Funktion eines Lösungsmittels zu. Ein solcher, Papier enthaltender Schichtkörper eignet sich besonders gut als Schall- und/oder Wärmedämmelement und hat gegenüber den bekannten Dämmelementen den Vorteil, daß er im noch feuchten Zustand besser geformt werden kann. Darüber hinaus kann das noch feuchte Element durch seinen eigenen inneren Zusammenhalt auch ohne Stützflächen gehandhabt werden, so ist es möglich, das noch feuchte Element senkrecht zu hängen, ohne daß die feuchte Papiermasse verrutscht. Ein feuchtes Element läßt sich auch um Träger oder dergleichen wickeln, an denen es seine endgültige Form schon vor dem Austrocknen erhält.

Mindestens auf eine der Oberflächen des feuchten, flächigen Schichtkörpers kann eine Prägung aufgebracht werden, es ist aber auch möglich, den gesamten Schichtkörper zu prägen, ohne, daß merkliche Unterschiede in der Wandstärke erhalten werden.

Ein weiteres mögliches Anwendungsgebiet kann dadurch erschlossen werden, daß als Kernschicht eine feuchte Rohkeramikmasse abgelegt wird. Durch das erfindungsgemäße Verfahren wird auch einer solchen, faserverstärkten Rohkeramikmasse ein innerer Zusammenhalt gegeben, so daß die Weiterverarbeitung von Rohkeramik erleichtert wird. Der noch feuchte Schichtkörper kann nach dem Vernadeln in einzelne Teile gewünschter Form, z. B. streifenförmig, kreisförmig, musterförmig geschnitten werden, woraufhin diese Teile verformt werden, ohne daß sie auseinanderfallen oder auseinanderreißen. Lange Streifen lassen sich z. B. spiralförmig, insbesondere um andere Gegenstände herum, wikkeln. So können z. B. elektrische Leitungen, insbesondere Heizdrähte, kontinuierlich umhüllt werden. Der Schichtkörper kann auch auf oder um eine »verlorene« Schalung gebracht werden, die, da auch ein erfindungsgemäß hergestellter Schichtkörper gebrannt werden kann, beim Brennen zerstört wird. Werden zum Vernadeln des Schichtkörpers synthetische Fasern verwendet, so pyrolisieren diese Fasern; diese Fasern haben somit für das fertige Produkt keine Bedeutung. Bei Verwendung von keramischen Fasern oder Steinwollfasern bleibt der Verstärkungseffekt der Fasern auch nach dem Brennen der Keramik erhalten.

Ausführungsbeispiele der Erfindung werden im folgenden anhand der Zeichnung und anhand von Beispielen erläutert. Es zeigt

Fig. 1 eine schematische Darstellung einer Anlage zur Durchführung des Verfahrens;

Fig. 2 eine schematische Darstellung des Schnittes durch einen vernadelten, noch feuchten Schichtkörper;

Fig. 3 eine schematische Darstellung eines Schnittes durch einen vernadelten und kalanderten Schichtkörper;

Fig. 4 einen Ausschnitt in Aufsicht auf einen mit Schlitzen versehenen Schichtkörper in schematischer Darstellung;

Fig. 5 und 6 zwei mögliche Ausbildungen von miteinander verbundenen Öffnungen, die bzw. deren Abschnitte zwischen sich Laschen bilden;

Fig. 7 einen Schichtkörper gemäß Fig. 5 und 6 im Schnitt, bei dem die Laschen aus der Ebene des Schichtkörpers herausgebogen sind, und

Fig. 8 einen flächigen Schichtkörper, auf den streifenförmige Schichtkörper aufgenadelt sind.

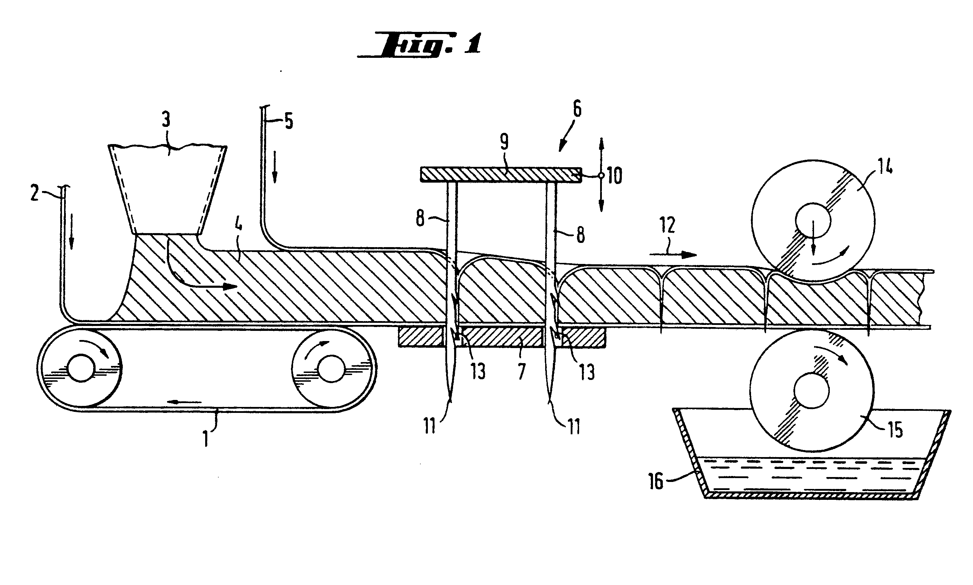

Gemäß Fig. 1 wird auf eine Fördereinrichtung, hier ein Förderband 1, eine Unterlagsschicht 2 abgelegt, auf die von einer Austragsvorrichtung 3 dosiert die Kernschicht 4 aufgetragen wird. Auf diese Kernschicht 4 werden aktiv nadelfähige Fasern, hier in Form eines Faservlieses 5 aufgelegt, worauf dieses Dreischichtensystem einer Nadelmaschine 6 zugeführt wird.

Solche Nadelmaschinen 6 sind aus der textilen Nadelfilztechnik bekannt (vgl. z. B. Krcma, Textilverbundstoffe, Seite 139 bis 141). Bei einer solchen Nadelmaschine 6 wird das zu vernadelnde System, hier das Dreischichtensystem, über eine mit Bohrungen versehene Grundplatte 7 geführt. Oberhalb des zu vernadelnden Gegenstandes ist ein die Vernadelungsnadeln 8 tragendes Nadelbrett 9 angeordnet, welches sich fortwährend so weit auf und ab bewegt (Doppelpfeil 10), daß die Nadelspitzen 11 in ihrer untersten Stellung den zu vernadelnden Gegenstand gewöhnlich ganz durchdrungen haben, während sie in ihrer obersten Stellung mit dem noch zu vernadelnden Gegenstand keine Berührung aufweisen. In dieser obersten Stellung kann der zu vernadelnde Gegenstand, hier das Dreischichtensystem, in Vorschubrichtung (Pfeil 12) taktweise verschoben werden, während es beim eigentlichen Vernadeln stillstehen muß. Die Vernadelungsnadeln 8 tragen an ihrem Schaft mindestens einen — hier zwei — Widerhaken 13, mit denen sie einzelne Fasern oder Faserbüschel ergreifen und in den zu vernadelnden Gegenstand hineinziehen, bzw. durch diesen hindurchziehen.

Beim Zurückfahren der Nadeln 8 lösen sich die mitgenommenen Fasern oder Faserbüschel von den Widerhaken 13 und verbleiben in der passiv vernadelten Schicht, hier der Unterlagsschicht 2 und der Kernschicht 4.

Während nun beim Vernadeln in der Textilindustrie, bei der Herstellung von Nadelfilzteppichen, die eine Enddicke von z. B. 4—6 mm aufweisen, die Nadelbretter 9 eine Vielzahl von dicht beieinander angeordneten Nadeln besitzen und dieses Nadelbrett z. B. mit einer Geschwindigkeit von 700 Hüben pro Minute bewegt werden kann, wird nun beim Vernadeln von Wasser enthaltenden Schichten, in denen auch noch Sandpartikel eingelagert sein können, die Dichte der Nadeln 8 in dem Nadelbrett 9 vergrößert und die Hubzahl stark verringert.

Sind diese Kriterien erfüllt und weist die noch feuchte Kernschicht die richtige Konsistenz auf, zu der noch Beispiele angeführt werden, so kann auch eine Schicht, die Rohkeramikmasse, Wasser getränkte Papierstreifen oder -schnitzel oder Moorbrei enthält, passiv vernadelt werden. Das Wasser wirkt dabei als Quell-, Schmier- und Gleitmittel, wodurch einerseits die Nadelspitzen leichter durch die aufgequollene Masse dringen können, andererseits die undurchdringbaren Partikel, wie z. B. Sandpartikel, innerhalb der Schicht geringfügig zur Seite ausweichen.

Wie aus Fig. 1 ersichtlich, verringert sich beim Vernadeln des Dreischichtensystems die Dicke desselben, da zum einen die Fasern enthaltende Schicht 5 durch das Vernadeln verdichtet wird, zum anderen diese Faserschicht 5 und, je nach Ausbildung, auch die Unterlagsschicht 2 in die Randbereiche der Kernschicht hineingedrückt werden.

Gemäß der in Fig. 1 dargestellten Ausführungsform der Anlage zum Durchführen des erfindungsgemäßen Verfahrens wird der vernadelte Schichtkörper zwischen zwei Kalanderwalzen 14 und 15 hindurchgeführt, die eine weitere Verdichtung des Schichtkörpers bewirken, wodurch insbesondere die in der Kernschicht enthaltene Luft und überschüssiges Wasser herausgepreßt werden. Zum Auffangen des überschüssigen Wassers ist hier eine Auffangwanne 16 vorgesehen, eine solche kann im übrigen auch unterhalb der Grundplatte 7 der Nadelmaschine 6 vorgesehen sein. Die beiden Kalanderwalzen 14 und 15 werden, zwischen sich den Schichtkörper führend, mit einem Druck von 2—5 bar aufeinander zugedrückt.

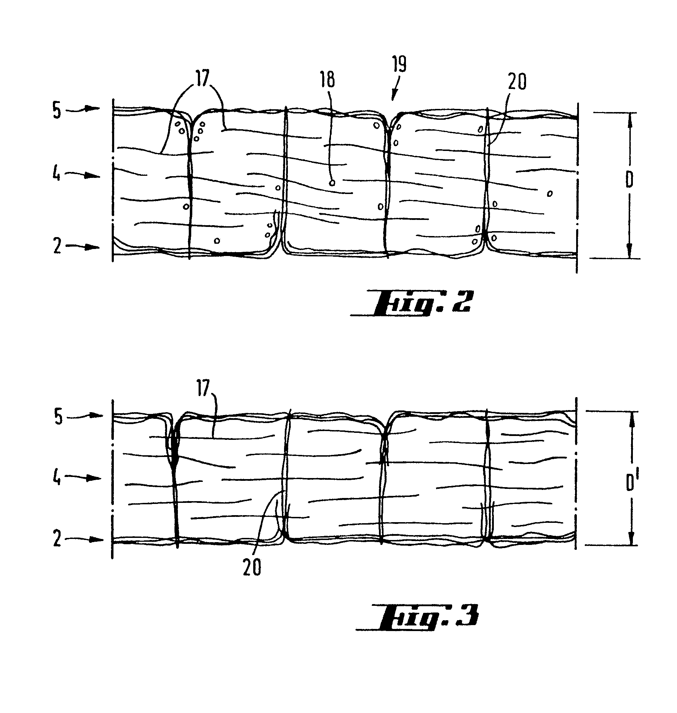

Die Fig. 2 und 3 zeigen in vergrößerter und schematischer Darstellung einen Schnitt durch einen vernadelten Schichtkörper, wobei Fig. 2 den Zustand nach dem Vernadeln, aber vor dem Kalandern und Fig. 3 den Zustand nach dem zusätzlichen Kalandern zeigen. Als Unterlagsschicht 2 wird gemäß den Fig. 2 und 3 ein aktiv vernadelbares Faservlies verwendet, das dem Faservlies 5 der Deckschicht entspricht. Die Kernschicht besteht hier — in den Fig. 2 und 3 — aus mit Wasser getränkten Papierstreifen und -schnitzeln 17, wobei dem Wasser vor dem Tränken noch Leim zugegeben wurde. Weiterhin sind der Fig. 2 noch einzelne Luftbläschen 18 entnehmbar, die sich insbesondere im Bereich der Einstichstellen der Vernadelungsnadeln 8 befinden. Im Bereich dieser Einstichstellen bilden sich auch »Fasertrichter« 19 aus. In diese Fasertrichter 19 werden auch Faserenden und Faserteile von Fasern, die nicht durch die Widerhaken 13 ergriffen werden, teilweise hineingezogen. Die den Schichtkörper durchdringenden Haltefasern 20 sind über die Fläche des Schichtkörpers ungleichmäßig verteilt, weshalb bei einem Schnitt durch einen solchen Schichtkörper in der Praxis nur sehr wenig Fasern zu sehen sind.

Wie schon oben ausgeführt, ist die Dicke D' nach dem Kalandern des Schichtkörpers (Fig. 3) geringer, als die Dicke D vor dem Kalandern desselben (Fig. 2). Weiterhin werden durch das Kalandern auch die Luftbläschen 18 entfernt.

Die Fig. 4 zeigt nun die Aufsicht auf einen vernadelten, noch feuchten Schichtkörper, der mit in parallelen Reihen angeordneten Schlitzen 22 versehen ist, wobei Schlitze benachbarter Reihen gegeneinander versetzt sind. Durch das Schlitzen erhält der Schichtkörper ein Dehnungsvermögen, wodurch er sich leicht Unebenheiten eines Gegenstandes, auf den er abgelegt wird, anpaßt.

Gemäß einem Ausführungsbeispiel der Erfindung weist ein Schichtkörper in seiner Kernschicht 4 nassen Moorbrei auf und wird nach dem Vernadeln mit Schlitzen versehen. Diese genadelte und geschlitzte Schichtkörperbahn wird in einzelne Flächengebilde, z. B. in quadratische Flächengebilde mit einer Seitenlänge von etwa 30 cm, aufgeteilt und kann dann als auf den menschlichen Körper aufzulegende Moorpackung verwendet werden. Durch das Dehnungsvermögen des Schichtkörpers paßt sich eine solche Moorpackung den Körperpartien, auf die sie aufgelegt wird, z. B. der Form des Gesichtes, an.

Gemäß einer speziellen Ausführungsform können in einer solchen Moorpackung einzelne Öffnungen für die Augen und die Nasenlöcher ausgestanzt werden. Die die drei Schichten verbindenden Haltefasern verhindern dabei ein Austreten der Moorpartikel.

Bei einer Ausführungsform der Moorpackung ist dem Moorbrei Sand beigegeben, wodurch sich diese Moorpackung zum Aufbereiten eines Moorbades eignet, in dem die Moorpackung in einer mit Wasser gefüllten Badewanne am Boden liegen bleibt.

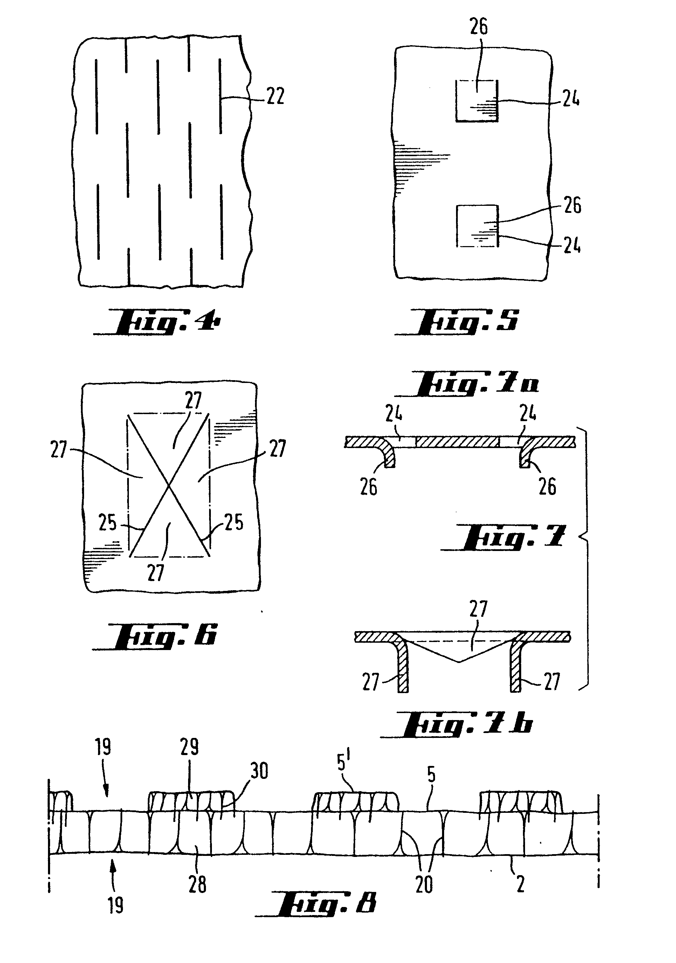

Bei der Ausführungsform des Schichtkörpers gemäß Fig. 5 sind in diesen U-förmige Schlitze eingebracht. Gemäß der Ausbildung nach Fig. 6 sind zwei sich kreuzende, ein »X« bildende Schlitze 25 vorgesehen. Zwischen den Schlitzen 24 bzw. den Abschnitten der Schlitze 25 verbleibt eine Lasche 26 bzw. verbleiben vier Laschen 27, die aus der Erstreckungsebene des Körpers in einem weiteren Verfahrensschritt herausgebogen werden, wie dies in Fig. 7 anhand eines Querschnitts durch den Körper dargestellt ist.

Anstelle des Schlitzens und darauf erfolgenden Herausbiegens der Laschen 26 und 27 werden gemäß einer anderen Ausführungsform des Verfahrens die Laschen in einem Arbeitsgang aus- und umgestanzt.

Fig. 8 zeigt nun einen ersten, flächigen Schichtkörper 28, auf den im noch feuchten Zustand weitere, hier streifenförmige, genadelte und noch feuchte Schichtkörper 29 aufgenadelt sind. Der flächige Schichtkörper 28 ist von beiden Seiten her vernadelt, was durch die angedeuteten Fasertrichter 19 und die Haltefasern 20 dargestellt ist. Der besseren Übersicht halber ist in Fig. 8 die zwischen der Unterlagsschicht 2 und der Deckschicht 5 befindliche Kernschicht, die z. B. der Kernschicht der Fig. 2 entspricht, nicht dargestellt. Die weiteren, streifenförmigen Schichtkörper 29 entsprechen in ihrem Aufbau dem flächigen Schichtkörper 28, sie weisen jedoch hier nur eine Dicke auf, die etwa der halben Dicke des Schichtkörpers 28 entspricht.

Diese streifenförmigen Schichtkörper 29 werden beabstandet voneinander auf den flächigen Schichtkörper 28 aufgelegt und dann mit diesem vernadelt, wobei die Vernadelungsnadeln von der Deckschicht 5' hereinstoßen, aus dieser Deckschicht 5' Haltefasern 30 mitnehmen und diese sowohl durch die Unterlagsschicht der streifenförmigen Schichtkörper 29, als auch durch die Deckschicht 5 des flächigen Schichtkörpers 28 in die Kernschicht des letzteren hineinstoßen.

Gemäß einer nicht dargestellten Ausführungsform werden nach dem anhand der Fig. 8 beschriebenen Verfahren auch zwei oder mehrere identische, flächige Schichtkörper 28 aufeinandergelegt und miteinander vernadelt. Durch mehrfaches Aufeinanderlegen von Schichtkörpern 28 und Vernadeln desselben kann ein Schichtkörper beliebiger Dicke hergestellt werden.

Anstelle der streifenförmigen Schichtkörper 29, die auf den flächenförmigen Schichtkörper 28 aufgenadelt werden, können auch Muster bildend Schichtkörper 29 mit anderen Flächen, z. B. kreisförmig oder quadratisch, aufgenadelt werden, je nachdem, welche Struktur das fertige Produkt aufweisen soll.

Die Zusammensetzung und der Aufbau einiger, nach dem erfindungsgemäßen Verfahren hergestellter Schichtkörper ergibt sich aus folgenden Beispielen.

### Beispiel 1

Zur Herstellung der Deck- und Unterlagsschicht wurde ein Vlies aus Polyesterfasern mit einem Flächengewicht von 80 g/m$^2$ auf einen Befatexträger mit einem Flächengewicht von 25 g/m$^2$ abgelegt. Es wurden hier verschiedene Polyesterfasern verwendet und zwar lag folgende Mischung vor: 30 g mit einem Titer von 4,4 dtex und einer Stapellänge von 100 mm, 30 g mit einem Titer von 6 dtex und einer Stapellänge von 60 mm und 20 g mit einem Titer von 15 dtex und einer Stapellänge von 76 mm. Dieses Vlies wurde mit dem passiv nadelfähigen Bafatexträger mit einer Stichdichte von 48 Stichen/cm$^2$ vorvernadelt.

Für die Kernschicht wurde ein Gemisch aus 2 Gewichtsteilen Portlandzement, 3 Gewichtsteilen Papierschnitzeln (Zeitungspapier) und 7 Gewichtsteilen Wasser verwendet. Dieses Gemisch wurde mit einem Flächengewicht von etwa 5,7 kg/m$^2$ zwischen die beiden Außenschichten gebracht, woraufhin das Dreischichtensystem in einer Nadelmaschine von beiden Seiten her vernadelt wurde. Die Stichdichte jeder Seite betrug 24 Stiche/cm$^2$. Der vernadelte Schichtkörper wurde 48 Stunden mit einem Druck von 40 N/cm$^2$ gepreßt, wobei die Presse während der ersten zwei Stunden Preßzeit auf 100°C erhitzt wurde und der Schichtkörper insgesamt 6 Tage austrocknete.

Man erhielt eine klopffeste Platte, deren beiden Oberflächen aus Fasern bestanden.

### Beispiel 2

Zur Herstellung der Deckschicht und der identischen Unterlagsschicht wurde ein Vlies aus 300 g Polypropylenfasern mit einem Titer von 17 dtex und einer Stapellänge von 90 mm auf ein Polypropylen-Bändchengewebe mit einem Flächengewicht von 80 g/m$^2$ vorvernadelt. Die Stichdichte beim Vorvernadeln betrug auch hier 48 Stiche/cm$^2$.

Als Kernschicht wurde hier ein Moorbrei verwendet, der auf 1 Gewichtsteil Feststoff, 7 Gewichtsteile Wasser enthält. Dieser Moorbrei wurde mit einem Flächengewicht von 10 kg/m$^2$ auf die vorvernadelte Unterlagsschicht gelegt und mit der Deckschicht abgedeckt. Das Dreischichtensystem wurde in einer Nadelmaschine von beiden Seiten her mit jeweils einer Stichdichte von 24 Stichen/cm$^2$ vernadelt. Es ergab sich eine Moorpackung mit einer Dicke von etwa 1 cm.

### Beispiel 3

Für die Unterlags- und die identische Deckschicht wurde ein Polyesterfasergemisch, 3 Gewichtsteile Fasern mit einem Titer von 4,4 dtex und 100 mm Stapellänge, 3 Gewichtsteile mit einem Titer von 6 dtex und einer Stapellänge von 60 mm und 2 Gewichtsteile mit einem Titer von 15 dtex und einer Stapellänge von 46 mm, mit einem gesamten Flächengewicht von 40 g/cm$^2$ vorvernadelt.

Auf die Unterlagsschicht wurde eine feuchte, verformbare Tonmasse mit einem Flächengewicht von 7 kg/m$^2$ aufgelegt, mit der Deckschicht bedeckt und von beiden Seiten her mit jeweils einer Stichdichte von 24 Stichen/cm$^2$ vernadelt.

Dieser vernadelte Schichtkörper wurde in 7 mm breite Streifen zerschnitten und spiralförmig um ein Rohr mit einem ∅ von 3 cm gewickelt. Diese aufgewickelte Spirale trocknete 24 Stunden bei Raumtemperatur aus und wurde dann von der Form genommen, innerhalb von zwei Stunden langsam auf 100°C erhitzt und dann 6 Stunden lang bei 1000°C gebrannt. In diesem Falle pyrolisierten die Polyesterfasern, sie wurden nur benutzt um die Schichtkörper bis zum Brennen einen inneren Zusammenhalt zu geben.

## Patentansprüche

1. Verfahren zur Herstellung von faserverstärkten, flächigen Körpern, bei dem eine Wasser enthaltende, fließfähige Kernschicht zwischen eine Unterlagsschicht und eine Deckschicht gebracht wird, dadurch gekennzeichnet, daß die drei Schichten, von denen mindestens eine äußere aus aktiv nadelfähigen Fasern besteht, im Wasser enthaltenden Zustand durch Vernadeln derart miteinander verbunden werden, daß die Schichten im verformbaren Zustand zusammenhalten.

2. Verfahren nach Anspruch 1, dadurch gekennzeichnet, daß die Schichten durch der Deckschicht und der Unterlagsschicht entnommene Haltefasern vernadelt werden.

3. Verfahren nach Anspruch 1 oder 2, dadurch gekennzeichnet, daß die Haltefasern unter einem Winkel von kleiner als 90° zur Erstreckungsebene des Körpers eingestoßen werden.

4. Verfahren nach Anspruch 3, dadurch gekennzeichnet, daß die Haltefasern sowohl von der Deckschicht als auch von der Unterlagsschicht her eingestoßen werden und zueinander windschief ausgerichtet sind.

5. Verfahren nach einem der vorhergehenden Ansprüche, dadurch gekennzeichnet, daß der vernadelte, noch feuchte Schichtkörper mit Öffnungen, vorzugsweise Schlitzen, versehen wird.

6. Verfahren nach Anspruch 5, dadurch gekennzeichnet, daß der Schichtkörper mit einer Vielzahl von in zueinander parallelen Reihen angeordneten Schlitzen versehen wird, wobei die Schlitze benachbarter Reihen gegeneinander versetzt angeordnet sind.

7. Verfahren nach Anspruch 5 oder 6, dadurch gekennzeichnet, daß der vernadelte und mit länglichen Öffnungen oder Schlitzen versehene Schichtkörper quer zur Erstreckungsrichtung der Öffnungen oder Schlitze gedehnt wird.

8. Verfahren nach einem der vorhergehenden Ansprüche, dadurch gekennzeichnet, daß der an sich ebene, noch feuchte Schichtkörper aus dieser Ebene heraus verformt wird.

9. Verfahren nach einem der vorhergehenden Ansprüche, dadurch gekennzeichnet, daß der noch feuchte Schichtkörper in einer Form tiefgezogen wird.

10. Verfahren nach einem der vorhergehenden Ansprüche, dadurch gekennzeichnet, daß der noch feuchte Schichtkörper gepreßt, insbesondere kalandert wird.

11. Verfahren nach einem der vorhergehenden Ansprüche, dadurch gekennzeichnet, daß zwei noch feuchte Schichtkörper durch Vernadeln miteinander verbunden werden.

12. Verfahren nach einem der Ansprüche 1 bis 10, dadurch gekennzeichnet, daß mindestens ein Schichtkörper flächig mit einem anderen, insbesondere flächigen Körper durch Vernadeln verbunden wird.

13. Verfahren nach einem der vorhergehenden Ansprüche, dadurch gekennzeichnet, daß der noch feuchte Schichtkörper, gegebenenfalls nach dem Formen bzw. Pressen, insbesondere unter Einwirkung von Hitze, getrocknet wird.

14. Verfahren nach einem der vorhergehenden Ansprüche, dadurch gekennzeichnet, daß die eine äußere Schicht, vorzugsweise die Unterlagsschicht, durch eine Kunststoff-Folie gebildet wird.

15. Verfahren nach einem der vorhergehenden Ansprüche, dadurch gekennzeichnet, daß eine medizinische, wasserenthaltende, natürliche Heilmasse als Kernschicht aufgelegt wird.

16. Verfahren nach Anspruch 15, dadurch gekennzeichnet, daß der nach dem Vernadeln in Form einer Bahn vorliegende Körper in einzelne Flächengebilde aufgeteilt wird und diese eine Heilpackung bildenden Flächengebilde luft- und wasserdicht verpackt werden.

17. Verfahren nach einem der Ansprüche 1 bis 12, dadurch gekennzeichnet, daß zerkleinertes Papier, insbesondere Altpapier, mit Wasser getränkt und als Kernschicht abgelegt wird.

18. Verfahren nach Anspruch 17, dadurch gekennzeichnet, daß vor dem Tränken dem Wasser ein Bindemittel beigefügt wird.

19. Verfahren nach Anspruch 17 oder 18, dadurch gekennzeichnet, daß der noch feuchte, flächige Schichtkörper mindestens auf einer seiner Oberflächen eine Prägung erhält.

20. Verfahren nach einem der Ansprüche 1 bis 12, dadurch gekennzeichnet, daß feuchte Rohkeramikmasse als Kernschicht abgelegt wird.

21. Verfahren nach Anspruch 20, dadurch gekennzeichnet, daß der noch feuchte Schichtkörper in Teile gewünschter Form, z. B. streifenförmig, kreisförmig, musterförmig, geschnitten wird.

22. Verfahren nach Anspruch 21, dadurch gekennzeichnet, daß die ausgeschnittenen Schichtkörperteile verformt, z. B. die Streifen spiralförmig gewickelt werden.

23. Verfahren nach einem der Ansprüche 20 bis 22, dadurch gekennzeichnet, daß der getrocknete und gegebenenfalls geformte Schichtkörper gebrannt wird.

## Claims

1. Method for producing fibre-reinforced flat bodies, wherein a water containing flowable core layer is introduced between a substrate layer and a cover layer, characterized in that the three layers of which at least one of the outer layers comprises actively needle bondable fibres, are bonded together in the water containing state by needling so that the layers have coherence in a deformable state.

2. Method according to Claim 1, characterized in that the layers are needle bonded by holding fibres taken from the cover layer and the substrate layer.

3. Method according to Claim 1 or 2, characterized in that the holding fibres are inserted at an angle of less than 90° with respect to the plane of extension of the body.

4. Method according to Claim 3, characterized in that the holding fibres are inserted both from the cover layer and the substrate layer and are skewed with respect to each other.

5. Method according to one of the foregoing Claims, characterized in that the needle bonded still wet composite body is provided with orifices, preferably slits.

6. Method according to Claim 5, characterized in that the composite body is provided with a plurality of slits arranged in parallel rows, with the slits of adjacent rows being offset with respect to each other.

7. Method according to Claim 5 or 6, characterized in that the needle bonded composite body provided with elongated orifices or slits is expanded transversely to the longitudinal direction of the orifices or slits.

8. Method according to one of the foregoing Claims, characterized in that the still wet composite body, flat in itself, is deformed from this plane.

9. Method according to one of the foregoing Claims, characterized in that the still wet composite body is deep drawn in a die.

10. Method according to one of the foregoing Claims, characterized in that the still wet composite body is pressed, in particular calendered.

11. Method according to one of the foregoing Claims, characterized in that two still wet composite bodies are needle bonded together.

12. Method according to one of the Claims 1 to 10, characterized in that at least one composite body is needle bonded to another, specifically flat body.

13. Method according to one of the foregoing Claims, characterized in that the still wet composite body is dried by the application of heat, if occasion arises after the forming respectively the pressing step.

14. Method according to one of the foregoing Claims, characterized in that one of the outer layers preferably the substrate layer consists of a sheet of synthetic plastic.

15. Method according to one of the foregoing Claims, characterized in that a medicinal, water containing, natural healing mass is introduced as the core layer.

16. Method according to Claim 15, characterized in that the composite body present after needle bonding in the form of a web is divided into individual flat aggregates and these flat aggregates representing a therapeutical pack, are packaged in an air and water tight manner.

17. Method according to one of the Claims 1 to 12, characterized in that shredded paper, especially waste paper, is soaked in water and introduced as the core layer.

18. Method according to Claim 17, characterized in that a binder is added to the water prior to the soaking.

19. Method according to Claim 17 or 18, characterized in that the still wet, planar composite body is embossed on at least one of its surfaces.

20. Method according to one of the Claims 1 to 12, characterized in that a wet raw ceramic mass is introduced as the core layer.

21. Method according to Claim 20, characterized in that the still wet composite body is cut into parts of a desired configuration, e. g. into strip or circular pieces or patterns.

22. Method according to Claim 21, characterized in that the cut parts of the composite body are deformed, e. g. the strips are wound in a helical manner.

23. Method according to one of the Claims 20 to 22, characterized in that the dried and if occasion arises formed composite body is baked.

**Revendications**

1. Procédé pour la fabrication de corps plats, renforcés de fibres, dans lequel une couche de noyau fluide, contenant de l'eau, est disposée entre une couche d'appui et une couche de couverture, caractérisé en ce que les trois couches, dont une extérieure au moins se compose de fibres activement aiguilletables, sont reliées l'une à l'autre par aiguilletage à l'état contenant de l'eau, de façon que les couches se maintiennent à l'état déformable.

2. Procédé selon la revendication 1, caractérisé en ce que les couches sont aiguilletées par des fibres de retenue prélevées de la couche de couverture et de la couche d'appui.

3. Procédé selon l'une quelconque des revendications 1 et 2, caractérisé en ce que les fibres de retenue sont enfoncées selon un angle inférieur à 90° par rapport au plan d'extension du corps.

4. Procédé selon la revendication 3, caractérisé en ce que les fibres de retenue sont enfoncées tant à partir de la couche de couverture que de la couche d'appui et sont alignées ensemble à l'état gauchi.

5. Procédé selon l'une quelconque des revendications 1 à 4, caractérisé en ce que le corps de couches aiguilleté, encore humide, est muni d'ouvertures, de préférence des fentes.

6. Procédé selon la revendication 5, caractérisé en ce que le corps de couches est muni d'un grand nombre de fentes disposées en rangées parallèles l'une à l'autre, les fentes des rangées voisines étant décalées réciproquement.

7. Procédé selon l'une quelconque des revendications 5 et 6, caractérisé en ce que le corps de couches aiguilleté et muni de fentes ou d'ouvertures oblongues est allongé transversalement au plan d'extension des ouvertures ou des fentes.

8. Procédé selon l'une quelconque des revendications 1 à 7, caractérisé en ce que le corps de couches plan, encore humide est déformé à par-

tir de ce plan.

9. Procédé selon l'une quelconque des revendications 1 à 8, caractérisé en ce que le corps de couches encore humide est embouti dans un moule.

10. Procédé selon l'une quelconque des revendications 1 à 9, caractérisé en ce que le corps de couches encore humide est pressé, en particulier calandré.

11. Procédé selon l'une quelconque des revendications 1 à 10, caractérisé en ce que deux corps de couches encore humides sont reliés l'un à l'autre par aiguilletage.

12. Procédé selon l'une quelconque des revendications 1 à 10, caractérisé en ce qu'au moins un corps de couches est relié à l'état plat, par aiguilletage, à un autre corps en particulier plat.

13. Procédé selon l'une quelconque des revendications 1 à 12, caractérisé en ce que le corps de couches encore humide est séché en particulier par l'action de la chaleur, éventuellement après le formage ou le pressage.

14. Procédé selon l'une quelconque des revendications 1 à 13, caractérisé en ce que la couche extérieure, de préférence la couche d'appui, est constituée d'une feuille de matière synthétique.

15. Procédé selon l'une quelconque des revendications 1 à 14, caractérisé en ce qu'une masse thérapeutique naturelle et médicale, contenant de l'eau, est utilisée comme couche de noyau.

16. Procédé selon la revendication 15, caractérisé en ce que le corps se présentant sous la forme d'une bande après l'aiguilletage est réparti en différents produits plats et en ce que ces produits plats formant un enveloppement thérapeutique sont emballés à l'état étanche à l'air et à l'eau.

17. Procédé selon l'une quelconque des revendications 1 à 12, caractérisé en ce que du papier fractionné, en particulier des vieux papiers, est imprégné d'eau et sert de couche de noyau.

18. Procédé selon la revendication 17, caractérisé en ce qu'un liant est ajouté à l'eau avant l'imprégnation.

19. Procédé selon l'une quelconque des revendications 17 et 18, caractérisé en ce que le corps de couches plat, encore humide reçoit un marquage au moins sur l'une de ses surfaces.

20. Procédés selon l'une quelconque des revendications 1 à 12, caractérisé en ce une masse de céramique brute humide sert de couche de noyau.

21. Procédé selon la revendication 20, caractérisé en ce que le corps de couches encore humide est découpé en parties d'une forme désirée, par exemple, sous la forme d'un ruban, sous la forme circulaire, sous la forme d'un modèle.

22. Procédé selon la revendication 21, caractérisé en ce que les parties découpées du corps de couches sont déformées, par exemple, les rubans sont enroulés en spirale.

23. Procédé selon l'une quelconque des revendications 20 à 22, caractérisé en ce que le corps de couches moulé le cas échéant et séché est brûlé.

**Fig. 1**

*Fig. 2*

*Fig. 3*

**Fig. 4**

**Fig. 5**

**Fig. 6**

**Fig. 7a**

**Fig. 7**

**Fig. 7b**

**Fig. 8**